# EUROPEAN PATENT SPECIFICATION

(11) **EP 1 463 513 B1**
(45) Date of publication and mention of the grant of the patent: **21.02.2007**
(21) Application number: 02804870.0
(22) Date of filing: 04.12.2002
(51) Int. Cl.: A61K 31/7072, A61K 47/08, A61K 47/10, A61K 47/12

(54) **STABILIZED BRIVUDINE TOPICAL FORMULATIONS**
STABILISIERTE TOPISCHE BRIVUDIN-FORMULIERUNGEN
PREPARATIONS TOPIQUES STABILISEES DE BRIVUDINE

(30) Priority: 19.12.2001 DE 10162593
(43) Date of publication of application: 06.10.2004
(73) Proprietor: Menarini Ricerche S.p.A., 00040 Pomezia (IT); BERLIN-CHEMIE Aktiengesellschaft, 12489 Berlin (DE)
(72) Inventor: SCHNITTKER, Christian, 12489 Berlin (DE); KEIPERT, Sigrid, 12489 Berlin (DE); GRÖGER, Karsten, 12489 Berlin (DE); SCHMITZ, Reinhard, 12489 Berlin (DE); MAGGI, Carlo, Alberto, I-00040 Pomezia (RM) (IT); MANZINI, Stefano, I-00040 Pomezia (RM) (IT)
(74) Representative: Banfi, Paolo
(86) International application number: PCT/EP2002/013714
(87) International publication number: WO 2003/051375

(56) References cited:
- EP-A- 0 755 675
- WO-A-98/10768
- DE-A- 19 956 601
- FREEMAN D J ET AL: "Preclinical assessment of topical treatments of herpes simplex virus infection: 5% (E)-5-(2-bromovinyl)-2'-deoxyuridine cream." ANTIVIRAL RESEARCH. NETHERLANDS JUN 1985, vol. 5, no. 3, June 1985 (1985-06), pages 169-177, XP008015919 ISSN: 0166-3542

## Description

The present invention relates to topical formulations containing the virustatic agent brivudine and stabilizers useful to prevent its photodegradation.

### State of the art

Brivudine ((E) -5-(2-bromovinyl)-2'-deoxyuridine) is an antiviral agent which is very effective against the varicella-zoster virus (VZV) and against the herpes simplex virus type 1 (HSV-1). Against VZV and HSV-1, brivudine is consistently more active than other known virustatic drugs such as aciclovir (ACV) and penciclovir (PCV). However, brivudine light-instability strongly limits its use in therapy. In particular, the UV light causes the isomerization of brivudine to Z-BVDU, as well as the formation of other decomposition products, with consequent decrease of clinical effectiveness. Photostabilization of brivudine is greatly recommended especially for dosage forms which are likely to undergo prolonged light exposure, such as dermal formulations.

Different approaches have been proposed to stabilize drugs from photodegradation.

US6136332 describes a non-aqueous dermatological/pharmaceutical composition for treating afflicted lips which is resistance to transfer or migration upon topical application to human skin. The resistance is the result of the combination of at least one volatile oil and at least one phenylated silicone oil. The invention also includes therapeutical agents having a degree of antiviral activity. Furthermore it includes organic and inorganic sunscreening agents but does not give any hint on how selecting the suitable ones. However, the invention does not resolve the problem of an effective photostabilization of the virustatic agent brivudine.

EP 0147811 describes the photostabilization of molsidomine by the addition of flavonoid derivatives, such as troxerutine, that exert an antioxidative effect.

US 5290774 describes the stabilization of light sensitive drugs by the use of polyhydric alcohols and boric acid, which form a complex in aqueous solution.

EP-A-755675 proposes to make topical compositions comprising an antiviral agent, such as BVDU.

DE 3136282 proposes to protect light-unstable drugs using substances with a similar absorption profile. The protective effect is supposed to depend on the similarity between the absorption spectra of the stabilizer and of the drug, that is, the larger is the overlapping between the two absorption spectra, the better is the photo-stabilization. According to DE3136282, the ICH (International Conference on Harmonisation) guideline CPMP/ICH/279/95 was followed to determine brivudine degradation. Several substances, including pharmaceutical excipients, colorants or photostable drugs were tested in admixture with brivudine. However, most of the tested food-colourants, including Quinoline yellow or Vanillin, did not satisfactorily stabilize brivudine.

### Description of the invention

It has now been found that certain substances with an absorption spectrum different from that of brivudine, provide a satisfactory photostabilization of the latter.

Best results were obtained with colourants of the azo-type, like Cochineal Red A (E 124; Ponceau 4R; 7-Hydroxy-8-(4-sulfonato-1-naphthylazo)-naphthalin-1,3-disulfonic acid, trisodium salt; CAS: 2611-82-7), Tropaeolin 0 (2,4-dihydroxyazobenzol-4'-sulfonic acid sodium salt; CAS: 547-57-9), or Food red 9 (E 123; 3-Hydroxy-4-(4-sulfonato-1-naphtylazo)-naphthalin-2,7- disulfonic acid, Trisodium salt; CAS: 915-67-3). This group of compounds is structurally characterized by the presence of an azo group in combination with two aromatic systems such as benzene, naphthalene, pyridine, pyrrole, thiophene, where the two aromatic systems may have up to four substituents selected from hydroxy, amino, sulfhydryl, methoxy, ethoxy, C1-C4-alkyl, carboxy, formyl, sulfonic or phosphonic acids and the corresponding salts.

The spectrum of these azo-colourants is notably different from that of brivudine. Cochineal Red A, for example, shows an absorption maximum at 330 and 505 nm, while the absorption maximum for brivudine is at 250 nm and 290 nm. Cochineal Red A led to a more then threefold increased photostabilization of brivudine. Food red 9 was found to protect brivudine from decomposition by more than 30% over the controls. The best recovery rates were obtained with Tropaelin 0. When used in a concentration of 5%, nearly no degradation of brivudine was detectable in a 1% formulation. Tropaeolin 0 shows an absorption maximum at about 395 nm.

In addition, several UV-absorbers were tested as photostabilizers for brivudine.

No effects were observed with padimate O, phenylbenzimidazole sulfonic acid, Uvinul® T150, Uvinul® P25 or homosalate.

Surprisingly UV-absorbers having an o-hydroxy-benzophenone structure, such as dioxybenzone, oxybenzone, 2,2',4,4'-tetrahydroxybenzophenone, 2,4-dihydroxy benzophenone, 2,2'-dihydroxy-4,4'-dimethoxybenzophenone-5,5'-disodium sulfonate, and sulisobenzone, were found to effectively stabilize brivudine formulations against UV-radiation. Although these absorbers have not an absorption profile similar to that of brivudine - for example the absorption maximum of 2,2',4,4'-tetrahydroxybenzophenon is about 350 nm -, they induced a threefold increased brivudine recovery after irradiation, i.e. about 75% of the initial concentration, with respect to brivudine alone. Benzophenone derivatives according to the invention include those compounds in which the aromatic rings forming the benzophenone moiety carry up to eight substituents selected from hydroxy-, amino, sulfhydryl-, methoxy-, ethoxy-, C1-C4-alkyl-, carboxy-, formyl-, sulfonic- and phosphonic acid and the corresponding salts. Included are also the same compounds bearing at least one hydroxy group in ortho position to the keto group of benzophenone.

Besides the o-hydroxy-benzophenone derivatives and the azo-colourants, photostabilization of brivudine was also obtained with carotenoids, especially with beta-carotene. The latter, which shows nearly no absorbance in the radiation range from 250 to 350nm, allows a remarkable photostabilization of brivudine. Carotenoids according to the invention refer to compounds having a tatraterpene structure with up to 12 conjugated double bonds selected from alpha-, beta-, gamma- and delta-carotene, lycopene, lutein, canthaxanthin, cryptoxanthin, rodoxanthin, zeaxanthin, crocetin, astaxanthin, fucoxanthin and violaxanthin.

Substances of the group of antioxidants/ radical quenchers were tested for their stabilization properties. Substances like cystein or alpha-liponic acid did not show any effect of brivudine photostabilization.

Differently, the UV-absorbers avobenzone, octocrylene, methylbenzylidene-camphor, octyl-methoxy-cinnamate, diphenyl-propane 1,3-dione derivatives and cyano-diphenyl acrylic acid derivatives were found to stabilize brivudine, although to a lesser extent than the benzophenone derivatives or the azo colourants. Therefore they were not deemed to be preferred solutions of the present invention.

According to the invention, the group of diphenyl-propane 1,3-dione derivatives includes compounds in which the two aromatic rings present in the diphenyl-propane 1,3-dione moiety bear up to six substituents independently selected from hydroxy, sulfhydryl, methoxy, ethoxy, C1-C4-alkyl, carboxy, formyl, sulfonic or phosphonic acid, and the corresponding salts, whereas cyano-diphenyl acrylic acid derivatives include those compounds in which the cyano-diphenyl acrylic acid moiety carries up to six substituents selected from hydroxy, sulfhydryl, methoxy, ethoxy, C1-C4-alkyl, carboxy, formyl, sulfonic or phosphonic acid, and the corresponding salts.

Object of the present invention is therefore a topical formulation containing brivudine as the active principle and at least one of the photostabilizer above mentioned, together with pharmaceutically acceptable excipients, according to claim 1.

The group of compounds having a phenyl-azo-benzene or an o-hydroxy-benzophenone structure, as well as the carotenoids, are preferred. UV-absorbers, especially those of the o-hydroxy-benzophenone type, allow the preparation of colorless dermal formulations, which guarantee an effective photostabilization of the drug once applied onto the skin. The efficacy of photostabilization can be further improved combining different o-hydroxy-benzophenone derivatives, UV-adsorbers, carotenoids and/or phenyl-azo-benzene derivatives. In fact, the hydrophilic/lipophilic balance of multicomponent formulations has been found to influence the effeciveness of photostabilization. For example, a combination of the hydrophilic and lipophilic photostabilizers sulisobenzone and, respectively, oxybenzone in a O/W emulsion, provides a better brivudine-photostabilization than the same concentration of each stabilizer separately.

According to the invention, the photo-stabilizers can be used in a concentration of 0.1% to 10%, preferably from 0.5 to 5% w/w.

Preferred topical formulations are in the form of gels, emulsions, microemulsions, creams, ointments and lipsticks. Suitable methods for preparing the pharmaceutical compositions of the invention are known to anyone skilled in the art and are described, for example, in Remington's Pharmaceutical Science, 17^{th} ed., Mack Publishing Company, Easton, PA (1985). For some formulations viscosity increasing ingredients were used. These are known thickening agents as derivatives of cellulose, starch and fatty acid as well as aluminium stearate, cetearyl alcohol, silica, beeswax, cera alba, Carbomer, polyvinyl alcohol. In any case no phenyl-containing silicone oil was used in the present formulations.
Where the formulation has not adequate antimicrobial activity per se, or it is not preserved by a special manufacturing or container, antimicrobial preservatives may be added, particularly for formulations in multidose containers. The antimicrobial preservatives can be selected from the group of quaternary ammonium compounds, for instance cetrimonium chloride or benzalkonium chloride, derivatives of the p-hydroxybenzoic acid, such as methyl-4-hydroxybenzoate or propyl-4-hydroxybenzoate, derivatives of benzoic acid and sorbic acid. Other antimicrobial preservatives are chorhexidine, chloroxymethylbenzole, imidazolidiny urea, 2-bromo-2-nitro-1,3-propanediol, benzyl alcohole, phenoxyethylalcohol, phenylmercuric acetate.

The stabilized formulations of the invention are conveniently used in the topical treatment of VZV and HSV-1 infections.

### Stability tests

**Table 1: Stabilization of brivudine by different substances in a tenside gel (brivudine-/dye-/UV-absorber-concentration: 1% w/w); Irradiation with a xenon lamp according to ISO 10977, SUNTEST CPS+, Atlas Co.**

| Dye/ UV absorber | % recovery rate (mean, n=2 to 13) |
|---|---|
| **no addition** | **26.1** |
| Quinoline Yellow* | 3.0 |
| Tartrazine* | 10.6 |
| beta carotene | 35.0 |
| Food red 9** | 56.2 |
| Cochineal Red A ** | 77.6 |
| Tropaeolin 0** | 84.9 |
| Sulisobenzone*** | 37.7 |
| 2,2',4,4'-tetrahydroxybenzophenon*** | 59.2 |
| Dioxybenzone*** | 69.3 |

| | |
|---|---|
| * negative examples (no photostabilizing effect in spite of similar absorption to brivudine) **azo-colourant ***benzophenon derivative | |

**Table 2: Stabilization of brivudine solutions by different dyes or UV-absorbers in a microemulsion (brivudine-/ dye-/ UV-absorber-concentration: 1% w/w); Irradiation with a xenon lamp, 7200kJ/m² according to ISO 10977, SUNTEST CPS+, Atlas Co.; because of a special sample presentation (sealed quartz containers) the recovery rates in the microemulsion are generally higher than those obtained with the tensid gel or the O/W creams**

| Dye/ UV absorber | recovery rate in% (mean, n=3) |
|---|---|
| **no addition** | **73.0** |
| Food red 9 ** | 99.1 |
| Cochineal Red A ** | 100.1 |
| Sulisobenzone*** | 88.1 |
| Oxybenzone*** | 89.6 |
| 2,2',4,4'-tetrahydroxybenzophcnon*** | 93.6 |
| Dioxybenzone*** | 94.4 |

| | |
|---|---|
| **azo-colourant ***benzophenon derivative | |

**Table 3: Photostabilization of brivudine by different UV-absorbers in O/W-cream (brivudine-concentration: 1% w/w; absorber-concentration 5% w/w); Irradiation with a xenon lamp, 7200kJ/m² according to ISO 10977, SUNTEST CPS+, Atlas Co.**

| UV absorber | recovery rate in % (mean, n=3) |
|---|---|
| **no addition** | **14.3** |
| Uvinul^{®} P25* | 0.3 |
| Padimate O | 17.2 |
| Sulisobenzone*** | 37.2 |
| Oxybenzone*** | 61.7 |
| 2,2',4,4'-tetrahydroxybenzophenon*** | 67.8 |
| Dioxybenzone*** | 85.7 |

| | |
|---|---|
| * negative examples (no photostabilizing effect) *** benzophenon derivative | |

**Table 4: Photostabilization of brivudine by different UV-absorbers in O/W-cream (brivudine-concentration: 2% w/w; absorber-concentration 5% w/w); Irradiation with a xenon lamp, 7200kJ/m² according to ISO 10977, SUNTEST CPS+, Atlas Co.**

| UV absorber | recovery rate in% (mean, n=3) |
|---|---|
| **no addition** | **9.9** |
| 2.5% Sulisobenzone***/ 2.5% Uvinul® D50*** | 48.1 |
| 2.5% Sulisobenzone***/ 2.5% Oxybenzone*** | 78.3 |
| 2.5% Sulisobenzone***/ 2.5% Dioxybenzone*** | 83 |

| | |
|---|---|
| ***benzophenon derivative | |

The following examples illustrate the invention in greater detail.

### EXAMPLES- Pharmaceutical compositions

The following microemulsions were prepared by mixing all ingredients at a temperature of 30°C for two hours:

### Example 1.

| | |
|---|---|
| brivudine | 1% |
| oxybenzone | 4% |
| isopropyl myristate | 29.5% |
| poloxamer 101 | 11.8% |
| polysorbate 85 | 17.7% |
| silicon dioxide | 10.7% |
| water | q.s. ad 100% |

An antimicrobial preservative can eventually be added.

### Example 2

| | |
|---|---|
| brivudine | 1 to 2.5% |
| dioxybenzone | 0.5 to 2.5% |
| isopropyl myristate | 25 to 37% |
| poloxamer 101 | 8 to 20% |
| polysorbate 85 | 15 to 25% |
| silica | 5 to 12% |
| methyl-4-hydroxybenzoate | 0.18% |
| propyl-4-hydroxybenzoate | 0.2% |
| water | q.s. ad 100% |

### Example 3

| | |
|---|---|
| brivudine | 1 to 2.5% |
| 2,2',4,4'-tetrahydroxybenzophenone | 0.5 to 5% |
| isopropyl palmitate | 25 to 37% |
| poloxamer 101 | 8 to 20% |
| PEG-(50)-sorbitol-hexaoleat | 15 to 25% |
| benzalkonium chloride | 0.05 to 0.1 % |
| edetic acid | 0.1% |
| water | q.s. ad 100% |

### Example 4

| | |
|---|---|
| brivudine | 1 to 2.5% |
| oxybenzone | 0.5 to 6% |
| isopropyl myristate | 25 to 37% |
| poloxamer 101 | 8 to 20% |
| polysorbate 85 | 15 to 25% |
| water | q.s. ad 100% |

### Example 5

| | |
|---|---|
| brivudine | 1 to 2.5% |
| sulisobenzone | 0.5 to 2.5% |
| isopropyl myristate | 25 to 37% |
| poloxamer 122 | 8 to 20% |
| PEG-(40)-sorbitol hexaoleate | 15 to 25% |
| sorbic acid | 0.1 to 0.2% |
| citric acid | q.s. |
| water | q.s. ad 100% |

### Example 6

| | |
|---|---|
| brivudine | 1 to 2.5% |
| cochineal Red A | 0.5 to 2.5 |
| isopropyl myristate | 25 to 37% |
| poloxamer 101 | 8 to 20% |
| PEG-(25)-Glycerin-Trioleate | 15 to 25% |

The following gel formulation was prepared by mixing the poloxamers and water at temperatures between 10°C to 20°C for 6 to 8 hours and then adding the remaining ingredients under intensive mixing:

### Example 7

| | |
|---|---|
| brivudine | 1 to 2% |
| dioxybenzone | 0.5 to 2.5% |
| poloxamer 407 | 1 to 10% |
| poloxamer 188 | 10 to 40% |
| water | q.s. ad 100% |

### Example 8

| | |
|---|---|
| brivudine | 1 to 2% |
| 2,2',4,4'-tetrahydroxybenzophenone | 0.5 to 5% |
| poloxamer 407 | 1 to 10% |
| poloxamer 188 | 10 to 40% |
| methyl-4-hydroxybenzoate | 0.18% |
| propyl-4-hydroxybenzoate | 0.02% |
| water | q.s. ad 100% |

### Example 9

| | |
|---|---|
| brivudine | 1 to 2% |
| sulisobenzone | 0.5 to 5% |
| sodium hydroxide | q.s. |
| poloxamer 407 | 1 to 10% |
| poloxamer 188 | 10 to 40% |
| chlorohexidine | 0.05% |
| water | q.s. ad 100% |

### Example 10

| | |
|---|---|
| brivudine | 1 to 2% |
| cochineal red A | 0.5 to 5% |
| poloxamer 407 | 1 to 10% |
| poloxamer 188 | 10 to 40% |
| cetrimonium chloride | 0.05 to 0.1 % |
| water | q.s. ad 100% |

### Example 11

| | |
|---|---|
| brivudine | 1 to 2% |
| beta carotene | 0.1 to 2.5% |
| poloxamer 407 | 1 to 10% |
| poloxamer 188 | 10 to 4% |
| methyl-4-hydroxybenzoate | 0.18% |
| propyl-4-hydroxybenzoate | 0.2% |
| water | q.s. ad 100% |

The following Carbomer gels are prepared by mixing all ingredients with exception of 1,2-propandiol and water. Afterwards the mixture is added to 1,2-propandiol. At least the water is added. This system is mixed until a clear gel is formed.

### Example 12

| | |
|---|---|
| brivudine | 1 to 2.5% |
| sulisobenzone | 0.5 to 2.5% |
| carbomer 940 | 1 to 2% |
| tris(hydroxymethyl)aminomethane | 1 to 3% |
| 1,2-propandiol | 5 to 40% |
| citric acid | q.s. |
| methyl-4-hydroxybenzoate | 0.18% |
| propyl-4-hydroxybenzoate | 0.2% |
| water | q.s. to 100% |

### Example 13

| | |
|---|---|
| brivudine | 1 to2.5% |
| cochineal red A | 0.5 to 2.5% |
| carbomer 940 | 1 to 2% |
| tris(hydroxymethyl)aminomethane | 1 to 3% |
| 1,2-propandiol | 5 to 40% |
| benzalkonium chloride | 0.1 % |
| edetic acid | 0.1% |
| water | q.s. to 100% |

### Example 14

The following **O/W creams** were prepared by emulsification and homogenisation at 80°C, cooling down to 20°C to 30°C and adding the active ingredient under intensive mixing.

| | |
|---|---|
| brivudine | 1 to 2.5% |
| sulisobenzone | 0.5 to 2.5% |
| oxybenzone | 0.1 to 6% |
| 1,2-propandiol | 10 to 20% |
| potassium hydroxide | q.s. |
| paraffin oil, subliquidum | 15 to 30% |
| vasilinum album | 15 to 27% |
| polyoxyethylene-monostearate | 2 to 5% |
| cetostearyl alcohol | 2 to 5% |
| polydimethylsiloxane | 0.01 to 0.5% |
| imidazolidiny urea | 0.6% |
| propyl-4-hydroxybenzoate | 0.1 to 0.2% |
| water | q.s. ad 100% |

### Example 15

The following ointment is prepared by melting and mixing the oily ingredients. Then the active ingredient and the UV-Absorber are added. The ointment is cooled down to room temperature maintaining stirring and homogenising if necessary.

| | |
|---|---|
| brivudine | 1 to 2.5% |
| oxybenzone | 0.5 to 6% |
| paraffin oil, perliquidum | 2 to 10% |
| vasilinum album | q.s. ad 100% |

The following lipsticks were prepared by melting and mixing the lip stick ingredients at about 70°C to 80°C. Then the active ingredient and the UV-Absorber are added. Subsequently the mixture is cast into lip stick container.

### Example 16

| | |
|---|---|
| brivudine | 1 to 2.5% |
| dioxybenzone | 0.5 to 2.5% |
| cochineal red A | 0.1 to 1% |
| glycerine monostearate | 6 to 10% |
| hydrogenated coco-glycerides | 28 to 29% |
| caprylic/ capric triglyceride | 38 to 39% |
| glycerine tricaprate | 4 to 8% |
| cera alba | 4 to 6% |
| hydrogenated palm oil | 1 to 3% |
| vaselinum album | q.s. to 100% |

### Example 17

| | |
|---|---|
| brivudine | 1 to 2.5% |
| oxybenzone | 0.5 to 6% |
| cochineal red A | 0.1 to 1% |
| glycerine monostearate | 12 to 20% |
| hydrogenated coco-glycerides | 28 to 29% |
| caprylic/ capric triglyceride | 28 to 39% |
| mixed ester of diglycerol with caprylic-/capric-/ isostearic-/hydroxystearic- and adipic acid | 13 to 34% |
| cera alba | q.s. to 100% |

### Description of the non-proprietary names used:

| **Substance** | **description** |
|---|---|
| 1,2-propandiol | Propylene Glycol (USP 24) |
| Carbomer 940 | Carbomer 940 (USPNF19) |
| Carbomer 934 P | Carbomer 934 P (USPNF 19) |
| Cera alba | White wax (USPNF 19) |
| Cetostearyl alcohol | Cetostearyl alcohol (USPNF 19) |
| Cochineal Red A | E 124; Ponceau 4R; 7-Hydroxy-8-(4-sulfonato-1-naphthylazo)-naphthalin-1,3-disulfonic acid, Trisodium salt; CAS: 2611-82-7 |
| Dioxybenzone | Dioxybenzone (USP24) |
| Phenylbenzimidazole Sulfonic Acid | Phenylbenzimidazole Sulfonic Acid (USP24) |
| Padimate O | Padimate O (USP24) |
| Homosalate | Homosalate (USP24) |
| Food red 9 | E 123; 3-Hydroxy-4-(4-sulfonato-1-naphtylazo)-naphthalin-2,7-disulfonic acid, Trisodium salt; CAS: 915-67-3 |
| Isopropyl Myristate | Isopropyl Myristate (USPNF19) |
| Poloxamer 188 | Poloxamer 188 (USPNF 19) |
| Poloxamer 407 | Poloxamer 407 (USPNF 19) |
| paraffin oil, perliquidum | Paraffinum liquidum (Ph. Eur.) |
| paraffin oil, subliquidum | Mineral oil (USP 24) |
| Polydimethylsiloxane | Dimethicone (USPNF 19) |
| Potassium hydroxide | Potassium hydroxide (USP24; Reagents) |
| Quinoline yellow | Type I: 2-(1,3-Dioxo-2-indanyl)-chinolin-disulfonic acid, Disodium salt Type II: 2-(1,3-Dioxo-2-indanyl)-methylchinolin-disulfonic acid, Disodium salt;CAS: 8004-92-0; |
| | E104 |
| Sodium hydroxide | Sodium hydroxide (USPNF19) |
| Tartrazine | 5-Hydroxy-1-(4-sulfonatophenyl)-4-(4-sulfonatophenylazo)-pyrazole-3-carbonic acid, Trisodiumsalt; CAS: 1934-21-0; E 102 |
| Tris(hydroxymethyl)aminomethane | Tromethamine (USP24;Reagents) |
| Tropaeolin 0 | 2,4-Dihydroxyazobenzol-4'-sulfonic acid Sodium salt; CAS: 547-57-9 |
| Polysorbate 85 | Polyoxyethylene 20 sorbitan trioleate CAS: 9005-70-3 |
| Oxybenzone | Oxybenzone (USP24) |
| Octyl Methoxycinnamate | Octyl Methoxycinnamate (USP24) |
| Sulisobenzone | Sulisobenzone (USP24) |
| 2,2',4,4'-tetrahydroxybenzophenone | CAS:131-55-5 |
| 4-Bis(polyethoxy)paraamino-benzoicacid-polyethoxyethylester | CAS: 113010-52-9 |
| 2,4,6-trianilino-p-(carbo-2'-ethylhexyl-1'-oxi)-1,3,5-triazin | CAS: 88122-99-0 |
| Vanillin | Vanillin (USPNF19) |
| Vasilinum album | Petrolatum (USP24) |

## Claims

1. A pharmaceutical composition for topical use containing (E)-5-(2-bromovinyl)-2'-deoxyuridine (brivudine) and a photostabilizer selected from the group consisting of carotenoids and derivatives of benzophenone and phenyl-azo-benzene.

2. A pharmaceutical composition according to claim 1, wherein the photostabilizer is an o-hydroxy-benzophenone derivative.

3. A pharmaceutical composition according to claim 2 in which said o-hydroxy-benzophenone derivative is selected from the group consisting of: dioxybenzone, oxybenzone, 2,2',4,4'-tetrahydroxybenzophenone, sulisobenzone, 2,4-dihydroxy benzophenone, 2,2'-dihydroxy-4;4'-dimethoxybenzophenone 5,5-disodium sulfonate.

4. A pharmaceutical composition according to claim 1, wherein the photostabilizer is a phenyl-azo-benzene-derivative.

5. A pharmaceutical composition according to claim 4, wherein said phenyl-azo-benzene derivative is selected from the group consisting of 7-Hydroxy-8-(4-sulfonato-1-naphthylazo)-naphthalin-1,3-disulfonic acid trisodium salt, (2,4-dihydroxyazobenzol)-4'-sulfonic acid sodium salt and 3-Hydroxy-4-(4-sulfonato-1-naphtylazo)-naphthalin-2,7-disulfonic acid trisodium salt.

6. A pharmaceutical composition according to claim 1, wherein the photostabilizer is a carotenoid.

7. A pharmaceutical composition according to claim 6, wherein said carotenoid is beta-carotene

8. A pharmaceutical composition according to any preceding claims, containing at least one photostabilizer.

9. A pharmaceutical composition according to any preceding claims, Wherein the concentration of the photostailizer ranges from 0.1 to 10% w/w.

10. A pharmaceutical composition according to claim 9, wherein said concentration is from 0.5 to 5% w/w.

11. A pharmaceutical composition according to any preceding claims, in the form of tenside gel, microemulsion, hydrogel, O/W cream, W/O cream lipstick, lipogel, ointment.

12. A pharmaceutical composition according to any preceding claims represented by a microemulsion containing: brivudine 1%; oxybenzone 4%; isopropyl myristate 29,5%; poloxamer 101, 11.8% polysorbate 85, 17.7%, silicon dioxide 10.7% eventually an antomicrobial preservative, water q.s. ad 100%.

13. A pharmaceutical composition according to any preceding claims, for the topical treatment of varicella zoster virus and herpes simplex type 1 virus infections.

## Patentansprüche

1. Pharmazeutische Zusammensetzung für die topische Verwendung, die (E)-5-(2-Bromovinyl)-2'-deoxyuridin (Brivudin) und einen Photostabilisator, ausgewählt aus der Gruppe bestehend aus Karotinoiden und Derivaten von Benzophenon und Phenylazobenzol, enthält.

2. Pharmazeutische Zusammensetzung nach Anspruch 1, wobei der Photostabilisator ein o-Hydroxybenzophenon-Derivat ist.

3. Pharmazeutische Zusammensetzung nach Anspruch 2, in welchem das o-Hydroxybenzophenon-Derivat ausgewählt ist aus der Gruppe bestehend aus Dioxybenzon, Oxybenzon, 2,2',4,4'-Tetrahydroxybenzophenon, Sulisobenzon, 2,4-Dihydroxybenzophenon, 2,2'-Dihydroxy-4,4'-dimethoxybenzophenon-5,5'-Dinatriumsulfonat.

4. Pharmazeutische Zusammensetzung nach Anspruch 1, wobei der Photostabilisator ein Phenyl-azo-benzol-Derivat ist.

5. Pharmazeutische Zusammensetzung nach Anspruch 4, wobei das Phenyl-azo-benzol-Derivat ausgewählt ist aus der Gruppe bestehend aus 7-Hydroxy-8-(4-sulfonato-1-naphthylazo)-naphthalin-1,3-Disulfonsäure-Trinatriumsalz, (2,4-Dihydroxyazobenzol)-4'-Sulfonsäure-Natriumsalz und 3-Hydroxy-4-(4-sulfonato-1-naphthylazo)-naphthalin-2,7-Disulfonsäure-Trinatriumsalz.

6. Pharmazeutische Zusammensetzung nach Anspruch 1, wobei der Photostabilisator ein Karotinoid ist.

7. Pharmazeutische Zusammensetzung nach Anspruch 6, wobei das Karotinoid beta-Karotin ist.

8. Pharmazeutische Zusammensetzung nach einem der vorhergehenden Ansprüche, die zumindest einen Photostabilisator enthält.

9. Pharmazeutische Zusammensetzung nach einem der vorhergehenden Ansprüche, in der die Konzentration des Photostabilisators in dem Bereich von 0,1 bis 10 Gew.-% liegt.

10. Pharmazeutische Zusammensetzung nach Anspruch 9, wobei die Konzentration 0,5 bis 5 Gew.-% beträgt.

11. Pharmazeutische Zusammensetzung nach einem der vorhergehenden Ansprüche in Form eines Tensid-Gels, einer Mikroemulsion, eines Hydrogels, einer O/W-Creme, einer W/O-Creme, eines Lippenstifts, eines Lipogels oder einer Salbe.

12. Pharmazeutische Zusammensetzung nach einem der vorhergehenden Ansprüche, die als Mikroemulsion vorliegt und enthält: 1 % Brivudin; 4 % Oxybenzon; 29,5 % Isopropylmyristat; 11,8 % Poloxamer 101; 17,7 % Polysorbat 85; 10,7 % Siliziumdioxid; schließlich ein mikrobielles Konservierungsmittel; q.s./ad 100% Wasser.

13. Pharmazeutische Zusammensetzung nach einem der vorhergehenden Ansprüche, für die topische Behandlung von Infektionen mit dem Varicella zoster-Virus und dem Herpes simplex Typ 1-Virus.

## Revendications

1. Composition pharmaceutique pour usage topique contenant de la (E)-5-(2-bromovinyl)-2'-désoxyuridine (brivudine) et un photo-stabilisateur choisi dans le groupe constitué des caroténoïdes et des dérivés de benzophénone et de phényl-azo-benzène.

2. Composition pharmaceutique selon la revendication 1, dans laquelle le photo-stabilisateur est un dérivé o-hydroxy-benzophénone.

3. Composition pharmaceutique selon la revendication 2, dans laquelle ledit dérivé o-hydroxy-benzophénone est choisi dans le groupe constitué de dioxybenzone, oxybenzone, 2,2',4,4'-tétrahydroxybenzophénone, sulisobenzone, 2,4-dihydroxy benzophénone, 2,2'-dihydroxy-4,4'diméthoxybenzophénone-5,5'-disodium sulfonate.

4. Composition pharmaceutique selon la revendication 1, dans laquelle le photo-stabilisateur est un dérivé phényl-azo-benzène.

5. Composition pharmaceutique selon la revendication 4, dans laquelle ledit dérivé phényl-azo-benzène est choisi dans le groupe constitué du sel trisodique de l'acide 7-Hydroxy-8- (4-sulfonato-1-naphthylazo)-naphthalin-1,3-disulfonique, du sel sodique de l'acide (2,4-dihydroxyazobenzol)-4'-sulfonique et du sel trisodique de l'acide 3-Hydroxy-4-(4-sulfonato-1-naphtylazo)-naphthalin-2,7-disulfonique.

6. Composition pharmaceutique selon la revendication 1, dans laquelle le photo-stabilisateur est un caroténoïde.

7. Composition pharmaceutique selon la revendication 6, dans laquelle ledit caroténoïde est le beta-carotène.

8. Composition pharmaceutique selon l'une quelconque des revendications précédentes, contenant au moins un photo-stabilisateur.

9. Composition pharmaceutique selon l'une quelconque des revendications précédentes, dans laquelle la concentration du photo-stabilisateur va de 0,1 à 10% p/p.

10. Composition pharmaceutique selon la revendication 9, dans laquelle ladite concentration va de 0,5 à 5% p/p.

11. Composition pharmaceutique selon l'une quelconque des revendications précédentes, sous forme d'un gel tenside, de microémulsion, hydrogel, crème H/E, crème E/H, bâton à lèvre, lipogel, pommade.

12. Composition pharmaceutique selon l'une quelconque des revendications précédentes, représentée par une microémulsion contenant : 1% de brivudine ; 4% d'oxybenzone; 29,5% de myristate d'isopropyle ; 11,8% de poloxamer 101 ; 17,7% de polysorbate 85 ; 10,7% de dioxyde de silicium ; éventuellement un conservateur antimicrobien ; eau q.s. ad 100%.

13. Composition pharmaceutique selon l'une quelconque des revendications précédentes, pour le traitement topique d'infections par le virus de la varicelle et du zona et par le virus herpès simplex de type 1.
